(19) Europäisches Patentamt
European Patent Office
Office européen des brevets

(11) **EP 1 715 457 B1**

(12) **EUROPEAN PATENT SPECIFICATION**

(45) Date of publication and mention
of the grant of the patent:
**25.07.2012 Bulletin 2012/30**

(51) Int Cl.:
***G06T 7/20*** *(2006.01)*       ***A61B 8/00*** *(2006.01)*

(21) Application number: **06005362.6**

(22) Date of filing: **16.03.2006**

(54) **Apparatus and method of estimating motion of a target object from a plurality of images**

Vorrichtung und Verfahren zur Schätzung der Bewegung eines Zielobjekts aus einer Vielzahl von Bildern

Appareil et procédé d'estimation du mouvement d'un objet cible à partir d'une pluralité d'images

(84) Designated Contracting States:
**DE FR IT NL**

(30) Priority: **20.04.2005 KR 2005032604**

(43) Date of publication of application:
**25.10.2006 Bulletin 2006/43**

(73) Proprietor: **SAMSUNG MEDISON CO., LTD.
Kangwon-do 250-875 (KR)**

(72) Inventor: **Shin, Dong Kuk
Nowon-gu
Seoul 139-929 (KR)**

(74) Representative: **Lorenz, Markus
Lorenz & Kollegen
Patentanwälte Partnerschaftsgesellschaft
Alte Ulmer Strasse 2
89522 Heidenheim (DE)**

(56) References cited:
**GB-A- 2 327 313     US-A1- 2002 167 537
US-B1- 6 192 156**

• **QINFEN ZHENG ET AL: "BALLOON MOTION
ESTIMATION USING TWO FRAMES",
PROCEEDINGS OF THE ASILOMAR
CONFERENCE ON SIGNALS, SYSTEMS AND
COMPUTERS. PACIFIC GROVE, NOV. 4 - 6, 1991;
[PROCEEDINGS OF THE ASILOMAR
CONFERENCE ON SIGNALS, SYSTEMS AND
COMPUTERS], LOS ALAMITOS, IEEE COMP.
SOC. PRESS, US, vol. 2 OF 02, 4 November 1991
(1991-11-04) , pages 1057-1061, XP000314508,**

**Description**

The present invention generally relates to an imaging system, and more particularly to an apparatus and method of estimating the motion of a target object from consecutively acquired images of an imaging system.

An imaging system is a system configured to display images of a target object and is widely used in various fields. An ultrasound diagnostic system is described below as an example of the imaging system.

The ultrasound diagnostic system projects ultrasound signal from the surface of a target object toward a desired portion within the target object and non-invasively obtains an ultrasound image of soft tissues or blood flow by using information obtained through ultrasound echo signals.

Compared to other medical imaging systems (e.g., X-ray diagnostic system, X-ray CT scanner, MRI and nuclear medicine diagnostic system), the ultrasound diagnostic system is advantageous since it is small in size and fairly inexpensive. Further, the ultrasound diagnostic system is capable of providing real-time display and is highly safe without dangerous side-effects such as exposure of X-rays, etc. Thus, it is extensively utilized for diagnosing the heart, abdomen and urinary organs, as well as being widely applied in the fields of obstetrics, gynecology, etc.

In particular, the ultrasound diagnostic system can form a panoramic ultrasound image based on ultrasound images, which are consecutively acquired by moving a probe along the surface of a human body. That is, the conventional ultrasound diagnostic system can form the panoramic ultrasound image by combining a currently acquired ultrasound image with previously acquired ultrasound images. For example, after consecutively acquiring ultrasound images of an object having an elongated shape (e.g., arm, leg, etc.) by moving the probe along a longitudinal direction of the object, the panoramic image can be formed by spatially combining the acquired ultrasound images. This makes it easy to observe the damaged portions of the object.

Generally, when displaying the panoramic ultrasound image or a moving ultrasound image, images estimating the motion of a target object are typically inserted between consecutive images in order to display the image that is close to a real image.

The conventional ultrasound diagnostic system estimates the motion of a target object by comparing all the pixels of consecutively inputted ultrasound images. Thus, a large amount of data needs to be processed, which typically requires a prolonged amount of time for such data to be processed.

Further, when the motion of a target object is estimated based on the consecutive ultrasound images, a speckle noise included in the ultrasound image may lessen the accuracy of such estimation.

Motion vector detection in a wavelet transformed video signal is disclosed in GB 2 327 313.

It is, therefore, an object of the present invention to provide an apparatus and method of decomposing consecutively inputted ultrasound images through wavelet transform to reduce the speckle noise and amount of data when estimating the motion of a target object based on the decomposed ultrasound images.

According to one aspect of the present invention, there is provided a method of estimating the motion of a target object from a plurality of ① images, including: a) selecting consecutive first and second images from the plurality of images; b) decomposing the first and second images into a plurality of sub-images based on the frequency components of the first and second images by n levels, respectively, wherein n is a positive integer; c) selecting first and second sub-images of low frequency components from the plurality of sub-images ②; d) setting a feature pixel in the second sub-image; e) selecting an image block containing the feature pixel and a predetermined number of neighborhood pixels of the feature pixel; f) selecting a reference region from the first sub-image by comparing the image block with the first sub-image; g) calculating displacements between pixels of the reference region and pixels of the image block; h) storing the calculated displacements; i) performing 1-level composition for the decomposed ③ images; j) repeatedly performing the steps c) to i) until the decomposed ③ images become 1-level decomposed images; and k) estimating the motion of the target object based on the stored displacements.

According to another aspect of the present invention, there is provided an apparatus of estimating the motion of a target object from a plurality of ① images, including: a first selecting unit for selecting consecutive first and second images from the plurality of images; a decomposing unit for decomposing the first and second images into a plurality of sub-images based on frequency components of first and second sub-images by n levels, wherein n is a positive integer; a second selecting unit for selecting the first and second sub-images of low frequency components from the plurality of sub-images ②; a setting unit for setting a feature pixel from pixels in the second sub-image; a third selecting unit for selecting an image block containing the feature pixel and a predetermined number of neighborhood pixels of the feature pixel; a fourth selecting unit for selecting a reference region from the first sub-image by comparing the image block with the first sub-image; a calculating unit for calculating displacements between pixels of the reference region and pixels of the image block; a storing unit for storing the calculated displacements; a composing unit for 1-level composing the decomposed ③ images; ④ and an estimation unit for estimating the motion of the target object based on the stored displacements.

The above and other objects and features of the present invention will become apparent from the following description of preferred embodiments given in conjunction with the accompanying drawings, in which:

Fig. 1 is a block diagram schematically illustrating an ultrasound diagnostic system constructed in accordance with the present invention;

Figs. 2A and 2B are flowcharts showing a motion estimating method performed in an image processor constructed in accordance with the present invention;

Fig. 3 is a schematic diagram showing a procedure of 1-level wavelet transform;

Fig. 4 is a schematic diagram showing sub-images obtained through wavelet transform;

Fig 5 is an exemplary diagram showing a procedure of 3-level wavelet transform;

Figs. 6A and 6B are diagrams showing a horizontal gradient filter and a vertical gradient filter, respectively;

Figs. 7A and 7B are schematic diagrams showing examples of applying a horizontal gradient filter and a vertical gradient filter to a sub-image; and

Fig. 8 is a schematic diagram showing an example of estimating the motion of a target object in accordance with the present invention.

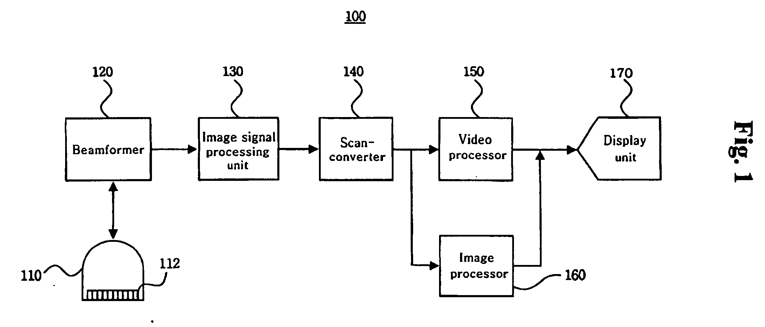

Fig. 1 is a block diagram schematically illustrating an ultrasound diagnostic system constructed in accordance with the present invention.

As shown in Fig. 1, an ultrasound diagnostic system 100 includes a probe 110, a beam-former 120, a signal processing unit 130, a scan converter 140, a video processor 150, an image processor 160 and a displaying unit 170. The ultrasound diagnostic system 100 may further include a storing unit such as a memory or the like. The video processor 150 and the image processor 160 may be provided as one processor.

The probe 110, which includes a 1-dimensional or 2-dimensional array transducer 112, is configured to sequentially transmit ultrasound signals to a target object as well as to sequentially receive echo signals from the target object. The ultrasound images of the target object may be consecutively acquired by scanning the target object with the probe 110. The consecutive ultrasound images may be a plurality of images displaying the motion of the target object. Also, the consecutive ultrasound images may be partial images of the target object. That is, an entire image of the target object may be observed through the partial images.

The beam-former 120 controls the delay of transmit signals, which are to be transmitted to the array transducer 112 of the probe 110 such that the ultrasound signals outputted from the array transducer 112 are focused on a focal point. It then focuses echo signals received by the array transducer 112 by considering the delay in which the echo signals reach each transducer.

The signal processing unit 130, which is a type of a digital signal processor, performs an envelope detection process for detecting the magnitude of the echo signal focused by the beam-former 120, thereby forming ultrasound image data.

The scan converter 140 performs the scan conversion for the ultrasound image data outputted from the signal processing unit 130.

The video processor 150 processes the scan-converted ultrasound image data outputted from the scan converter 140 to obtain a video format and transmit the processed ultrasound image data to the displaying unit 170.

The image processor 160 receives the ultrasound image data outputted from the scan converter 130 or the video processor 150.

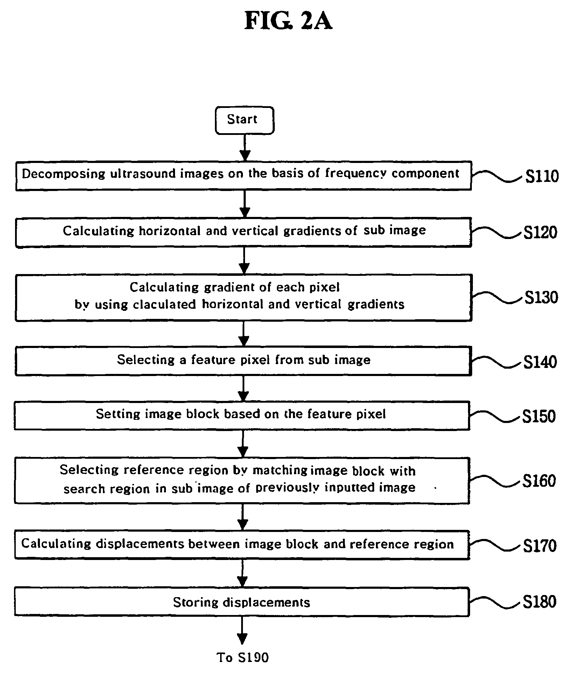

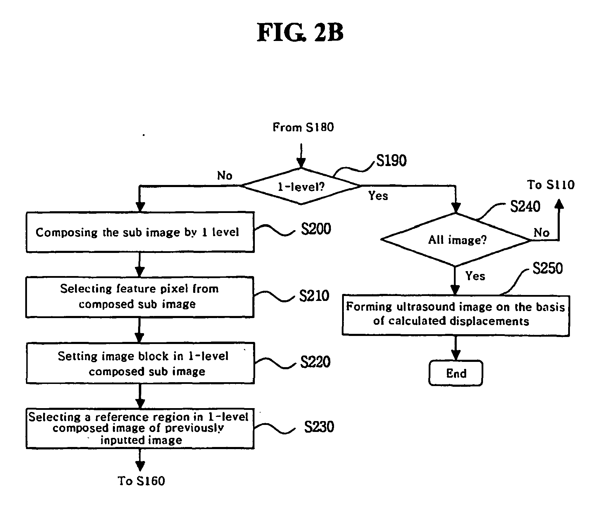

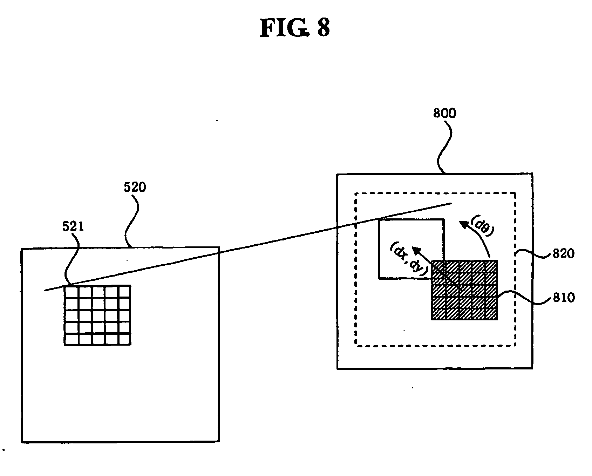

The operation of the image processor 160 is described below in view of Figs. 2 to 8.

Figs. 2A and 2B are flowcharts showing a motion estimating method, which is performed in the image processor 160 of the ultrasound diagnostic system 100.

Referring to Figs. 2A and 2B, the image processor 160 decomposes the ultrasound images, which are consecutively inputted from the scan converter 140, based on the frequency components of a predetermined number at step S110. An image acquired through n-level decomposition is referred to as an n-level image. According to the preferred embodiment of the present invention, wavelet transform may be used to decompose the ultrasound image.

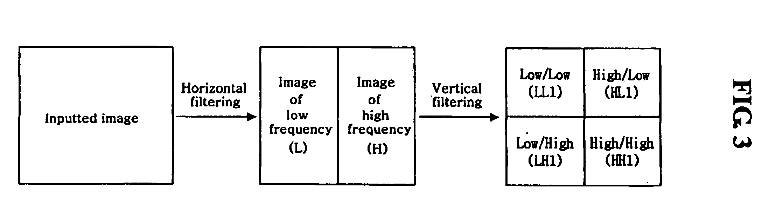

As illustrated in Fig. 3, a low pass filter and a high pass filter are applied to the inputted ultrasound image along a horizontal direction, thereby producing an image of a low band (L) and an image of a high band (H), respectively. The low pass filter and the high pass filter are then applied to L and H along a vertical direction, respectively, so that LL1, LH1, HL1 and HH1 sub-images can be obtained. The 1-level wavelet transform is carried out in accordance with the above process.

Thereafter, the low pass filter and the high pass filter are applied to the LL1 sub-image of a low frequency component along a horizontal direction. Then, the low pass filter and the high pass filter are applied along a vertical direction so that LL2, LH2, HL2 and HH2 sub-images can be obtained from the LL1 sub-image. As such, the 2-level wavelet transform can be completed. The 3-level wavelet transform is carried out for the LL2 sub-image of a low frequency component among the LL2, LH2, HL2 and HH2 sub-images.

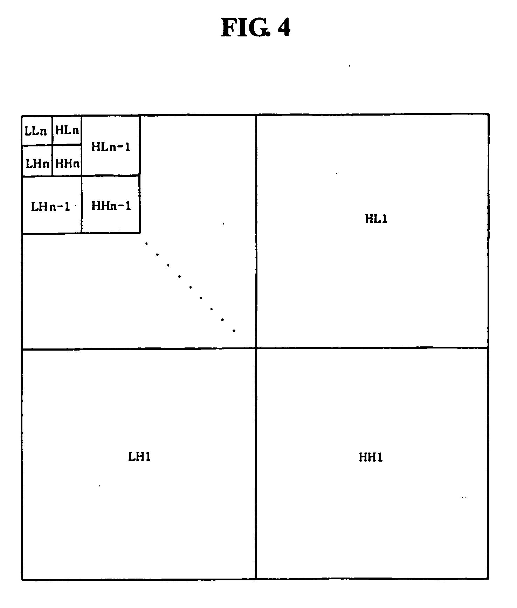

The image processor 160 continuously carries out the wavelet transform for the consecutively inputted ultrasound images of a predetermined number, thereby decomposing each ultrasound image into a plurality of sub-images to form and provide multi-resolution, as shown in Fig. 4. An LLn sub-image is the filtered original image of a low frequency component obtained through the wavelet transform. Further, HLn, LHn and HHn sub-images are images containing high frequency components of horizontal, vertical and diagonal orientations, respectively, for the LLn sub-image, wherein, n in LLn, HLn, LHn and HHn represents the level of wavelet transform.

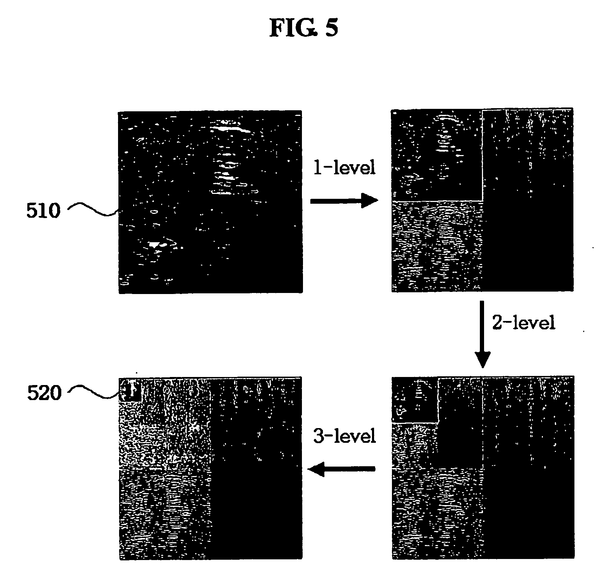

Fig. 5 illustrates the procedure of the 3-level wavelet transform for an inputted ultrasound image 510. An LL3 sub-image 520, which is shown in Fig. 5, is an image of a low frequency obtained through the 3-level wavelet transform.

The feature pixel is selected from the LL3 sub-image 520 obtained through the 3-level wavelet transform. The feature pixel is used as a reference pixel for motion estimation in accordance with the present invention. The feature pixel may be selected as a pixel having the highest gray level, luminescence or gradient among various pixels consisting the LL3 sub-image 520.

The reason for selecting the feature pixel from the LL3 sub-image 520 is that the LL3 sub-image 520 is an image, which can best remove the speckle noise among the HL3, LH3 and HH3 sub-images.

The method of selecting the feature pixel from the LL3 sub-image 520 is discussed below.

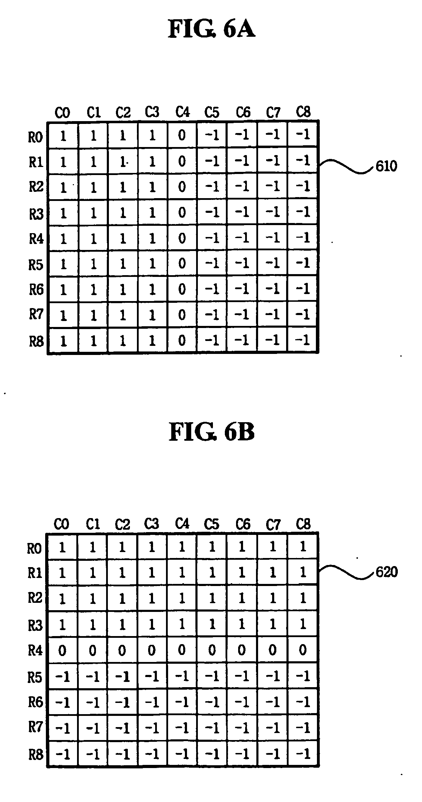

A horizontal gradient filter 610 and a vertical gradient filter 620 shown in Figs. 6A and 6B, respectively, are applied to each pixel comprising the LL3 sub-image 510. This is so that the horizontal and vertical gradients Sx and Sy of each pixel can be calculated at step S 120.

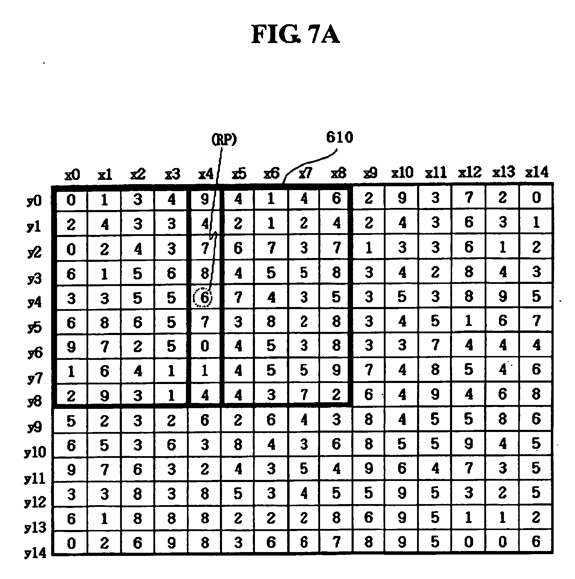

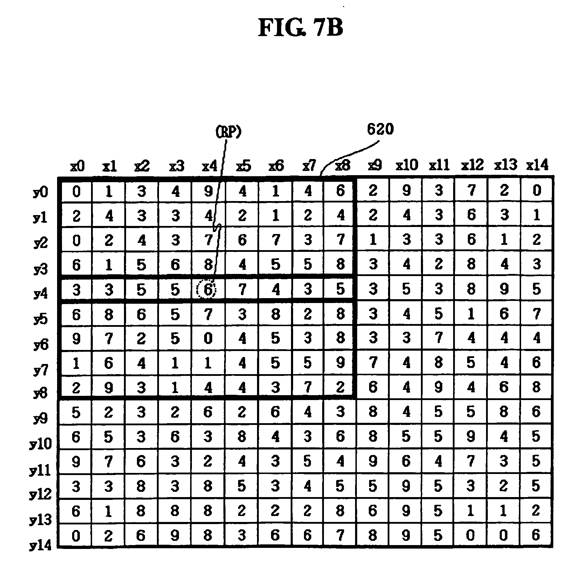

To calculate the horizontal gradient Sx of a target pixel RP included in the LL3 sub-image 510, which has a gray level distribution pattern as shown in Figs. 7A and 7B, the horizontal gradient filter 610 is applied to the LL3 sub-image 510. This is so that a center pixel (R4, C4) of the horizontal gradient filter 610 is positioned to the target pixel RP of the LL3 sub-image 520, as shown in Fig. 7A. Then, each pixel value of the horizontal gradient filter 610 is multiplied by each gray level of the LL3 sub-image 520. As such, the filtered values for the pixels of the LL3 sub-image 520 can be obtained.

Subsequently, the filtered values existing in left columns of center column x4 are summed together to thereby obtain a first adding value. Then the filtered values existing in right columns of center column x4 are also summed together, thereby obtaining a second adding value. That is, the first adding value is 138 (=0 + 2 + 0 + 6 + 3 + 6 + 9 + 1 + 2 + 1 + 4 + 2 + 1 + 3 + 8 + 7 + 6 + 9 + 3 + 3 + 4 + 5 + 5 + 6 + 2 + 4 + 3 + 4 + 3 + 3 + 6 + 5 + 5 + 5 + 1 + 10) and the second adding value is -168 (=-4 + (- 2) +(-6)+(-4)+(-7)+(-3)+(-4)+(-4)+(-4)+(-1)+(-1)+(-7)+(-5)+(-4)+ (-8)+(-5)+(-5)+(-3)+(-4)+(-2)+(-3)+(-5)+(-3)+(-2)+(-3)+(-5)+(-7)+(-6)+(-4)+(-7)+(-8)+(-5)+(-8)+(-8)+(-9)+(-2)).

The image processor 160 calculates the horizontal gradient Sx of the target pixel RP by summing the first adding value and the second adding value. The gradient of the target pixel RP becomes -30 (=138 + (-168)). The above process, which calculates the gradient of the target pixel RP is repeatedly carried out by changing the target pixel. The method of calculating a vertical gradient Sy of each pixel is carried out in a similar manner as the method of calculating the horizontal gradient Sx. In order to calculate the vertical gradient Sy, the vertical gradient filter 620 is applied to the LL3 sub-image 520 instead of the horizontal gradient filter 610, as shown in Fig. 7B.

After obtaining the filtered value by applying the vertical gradient filter 620 to the LL3 sub-image 520, the filtered values corresponding to coordinates (xi, yj) positioned at the upper side of center row y4 are summed together, wherein "i" is a positive integer ranging from 0 to 8 and "j" is a positive integer ranging from 0 to 3, thereby obtaining a third adding value. Also, the filtered values corresponding to coordinates (xm, yn) positioned at the lower side of center row y4 are also summed together, wherein "m" is a positive integer ranging from 0 to 8 and "n" is a positive integer ranging from 5 to 8, thereby obtaining a fourth adding value. Thereafter, the vertical gradient Sy of the target pixel RP is calculated by summing the third adding value and the fourth adding value. The above process, which calculates the vertical gradient of the target pixel RP, is repeatedly carried out by changing the target pixel.

After calculating the horizontal and vertical gradients Sx and Sy of each pixel as described above, the gradient S of each pixel is calculated by applying the calculated horizontal and vertical gradients Sx and Sy to the following equation:

$$S = \sqrt{S_x^2 + S_y^2} \qquad\qquad (1)$$

The calculated gradients of the pixels are compared with each other and the pixel having the maximum gradient is selected as the feature pixel at step S 140. If the feature pixel is selected, an image block 521 including the feature pixel and a predetermined number of pixels neighboring the feature pixel in the LL3 sub-image 520 is set at step S150.

After setting the image block 521, a reference region 810 is selected within a search region 820 of a LL3 sub-image 800, which is obtained from a immediately previous inputted ultrasound image through the 3-level wavelet transform, by using the image block 521 at step S160. The selection of the reference region 810 is determined based on a minimum sum absolute difference (SAD) by comparing the image block 521 with search the region 820.

The search region 820 is determined by removing the edge regions from the LL3 sub-image 800 in accordance with the preferred embodiment of the present invention. Also, the entire region of the LL3 sub-image 800 may be the search region 820 according to a process condition. Even if a region having the minimum SAD is searched in the edge region, the reference region should be selected from the region except the edge region.

The region having the minimum SAD may be searched based on the following equation:

$$\sum_{y=0}^{S}\sum_{x=0}^{S} | Pn_{(X-x-dx,Y-y-dy)} - Po_{(X-y,Y-y)} | \qquad (2)$$

Wherein Pn is a gray level of each pixel consisting the image block 521, Po is a gray level of each pixel consisting the previous LL3 sub-image 800, X and Y represent the coordinates of the feature pixel selected from current LL3 sub-image 520, and dx and dy are the distance displacements of coordinates between each pixel of the image block 521 and each pixel of the reference region 810.

The reference region 810, which is the region most corresponding to the image block 521, can be estimated as a region in which an image corresponding to the image block 521 is previously located. That is, it can be estimated that the image block 521 is moved from the reference region 810 in the previous LL3 sub-image 800. Accordingly, after calculating the displacements dx3 and dy3 of coordinates between each pixel of the reference region 810 and each pixel of the image block 520, the rotation displacement dq3 of coordinates, in which SAD becomes minimal, is calculated at step S170. In displacements dx3, dy3 and dq3, the number "3" means that the displacement is calculated for the 3-level wavelet transformed ultrasound image. Thereafter, it is determined whether the current image corresponds to a 1-level wavelet transformed image at step S 190.

If it is determined that the current image does not correspond to the 1-level wavelet transformed image at step S190, 1-level inverse wavelet transform is carried out at step S200. The image processor 160 determines the feature pixel from the sub-image of a low frequency component obtained through the 1-level inverse wavelet transform at step S210 and selects an image block based on the determined feature pixel at step S220. A region having the minimum SAD is selected as a reference region from the previous LL sub-image obtained through the 1-level inverse wavelet transform at step S230 and then the process proceeds to step S 170. That is, if the reference region is determined at step S230, displacements dx2, dy2 and dq2 are calculated by comparing the coordinates between each pixel of the reference region and each pixel of the image block at step S170. The calculated displacements dx2, dy2 and dq2 are stored at step S180. As described above, the number "2" in the displacements dx2, dy2 and dq2 means that the displacements are calculated for a 2-level wavelet transformed ultrasound image. Also, the displacements obtained through twice 1-level inverse wavelet transform can be represented as dx1, dy1 and dq1.

When the inverse wavelet transform is carried out, the size of the sub-image becomes enlarged. Therefore, the displacements are calculated in consideration that the sizes of the image block and the search region become enlarged as the inverse wavelet transform is carried out.

Meanwhile, if it is determined that the sub-images correspond to a 1-level wavelet transformed image at step S 190, then it is determined whether the above process (steps S110-S180) is carried out for all the ultrasound images in the image processor 160 at step S240. If it is determined that the above process is not carried out for all of the ultrasound images at step S240, then the process returns to step S 100 and then the process mentioned above is carried out. On the other hand, if it is determined that the process is completed for the all of the ultrasound images at step S240, the image processor 160 estimates the motion of the target object based on the displacements dx, dy and dq and then forms an ultrasound moving image at step S250. For example, the motion of the target object in an x direction can be estimated through selecting the smallest one among displacements dx3, dx2 and dx1 or averaging the displacements dx3, dx2 and dx1. The ultrasound moving image may be a typical moving image or a panoramic image.

As discussed above, since the consecutively inputted ultrasound images are wavelet transformed, the speckle noise can be reduced. Thus, the motion estimation of the target object in the ultrasound images is accurately carried out in accordance with the present invention. Also, since the decomposed image is used to estimate the motion of the target object, the amount of data to be processed for the motion estimation can be reduced. Therefore, the process of estimating the motion of the target object can be carried out more quickly.

While the present invention has been described and illustrated with respect to a preferred embodiment of the invention, it will be apparent to those skilled in the art that variations and modifications are possible without deviating from the scope of the claims appended hereto.

**Claims**

1. A method of estimating a motion of a target object from a plurality of consecutively inputted images, comprising:

   a) selecting consecutive first and second images from the plurality of images;

b) decomposing the first and second images into a plurality of sub-images based on the frequency components of the first and second images by n levels, respectively, wherein n is a positive integer;

c) selecting first and second sub-images of low frequency components from the plurality of sub-image corresponding to the first and second images;

d) setting a feature pixel in the second sub-image;

e) selecting an image block containing the feature pixel and a predetermined number of neighborhood pixels of the feature pixel;

f) selecting a reference region from the first sub-image by comparing the image block with the first sub-image;

g) calculating displacements between pixels of the reference region and pixels of the image block;

h) storing the calculated displacements;

i) performing 1-level composition for the decomposed first and second images,

j) repeatedly performing the steps c) to i) until the decomposed first and second images become 1-level decomposed images; and

k) estimating the motion of the target object based on the stored displacements.

2. The method as recited in claim 1, wherein the plurality of consecutive images are ultrasound images.

3. The method as recited in claim 1, wherein the steps b) and i) are carried out with wavelet transform and inverse wavelet transform, respectively.

4. The method as recited in claim 1, wherein the step d) comprises the steps of:

   d1) calculating horizontal and vertical gradients of each pixel comprising the second sub-image;
   d2) calculating gradient of each pixel of the second sub-image; and
   d3) comparing gradients of pixels with each other and selecting a pixel having maximal gradient as the feature pixel.

5. The method as recited in claim 4, wherein the horizontal and vertical gradients are calculated by using gray level of each pixel.

6. The method as recited in claim 4, wherein the horizontal and vertical gradients are calculated by using luminescence of each pixel.

7. The method as recited in claim 4, wherein the reference region has a minimum sum absolute difference with the image block.

8. The method as recited in claim 1, wherein the step g) comprises the steps of:

   g1) calculating distance displacements between each pixel of the image block and each pixel of the reference region; and
   g2) calculating rotation displacement by rotating the image block for the reference region.

9. An apparatus for estimating a motion of a target object from a plurality of consecutively inputted images, comprising:

   a first selecting unit for selecting consecutive first and second images from the plurality of images;
   a decomposing unit for decomposing the first and second images into a plurality of sub-images based on the frequency components of the first and second images by n levels, respectively, wherein n is a positive integer;
   a second selecting unit for selecting first and second sub-images of low frequency components from the plurality of sub-images corresponding to the first and second images;
   a setting unit for setting a feature pixel from pixels in the second sub-image;
   a third selecting unit for selecting an image block containing the feature pixel and a predetermined number of neighborhood pixels of the feature pixel;
   a fourth selecting unit for selecting a reference region from the first sub-image by comparing the image block with the first sub-image;
   a calculating unit for calculating displacements between pixels of the reference region and pixels of the image block;
   a storing unit for storing the calculated displacements;
   a composing unit for 1-level composing the decomposed first and second images a unit for repeatedly utilizing the second selecting unit, the setting unit, the third selecting unit, the found selecting unit, the calculating unit, the storing unit and the composing unit until the decomposed first and second images become 1-level decomposed images; and

an estimating unit for estimating the motion of the target object based on the stored displacements.

10. The apparatus as recited in claim 9, wherein the plurality of consecutive images are ultrasound images.

11. The apparatus as recited in claim 9, wherein the decomposing unit and the composing unit are operated by using wavelet transform and inverse wavelet transform, respectively.

12. The apparatus as recited in claim 9, wherein the setting unit includes:

a first calculating unit for calculating horizontal and vertical gradients of each pixel comprising the second sub-image;

a second calculating unit for calculating gradient of each pixel of the second sub-image by using the calculated vertical and horizontal gradients; and

a comparing unit for comparing gradients of pixels with each other and selecting a pixel having maximal gradient as the feature pixel.

13. The apparatus as recited in claim 12, wherein the horizontal and vertical gradients are calculated by using gray level of each pixel.

14. The apparatus as recited in claim 12, wherein the horizontal and vertical gradients are calculated by using luminescence of each pixel.

15. The apparatus as recited in claim 9, wherein the reference region has a minimum sum absolute difference with the image block.

16. The apparatus as recited in claim 9, wherein the calculating unit includes:

a first calculating unit for calculating distance displacements of coordinates between each pixel of the image block and each pixel of the reference region; and

a second calculating unit for calculating rotation displacement of coordinates by rotating the image block for the reference region.

**Patentansprüche**

1. Verfahren zur Schätzung einer Bewegung eines Zielobjekts aus einer Vielzahl von nacheinander eingegebenen Bildern, welches Folgendes aufweist:

a) Auswählen aufeinanderfolgender erster und zweiter Bilder aus der Vielzahl von Bildern;

b) Zerlegen der ersten und zweiten Bilder in eine Vielzahl von Unterbilder basierend auf den Frequenzkomponenten der ersten und zweiten Bilder jeweils in n Stufen, wobei n eine positive ganze Zahl ist;

c) Auswählen von ersten und zweiten Unterbildern mit niedrigen Frequenzkomponenten aus der Vielzahl von Unterbildern entsprechend den ersten und zweiten Bildern;

d) Festlegen eines Merkmalspixels in dem zweiten Unterbild;

e) Auswählen eines Bildblocks, welcher das Merkmalspixel und eine vorbestimmte Anzahl von zu dem Merkmalspixel benachbarten Pixels beinhaltet,

f) Auswählen eines Referenzbereichs aus dem ersten Unterbild durch Vergleichen des Bildblocks mit dem ersten Unterbild;

g) Berechnen von Verschiebungen zwischen Pixeln des Referenzbereichs und Pixeln des Bildblocks;

h) Speichern der berechneten Verschiebungen;

i) Durchführen einer 1-Level-Verknüpfung für die zerlegten ersten und zweiten Bilder;

j) wiederholtes Durchführen der Schritte c) bis i) bis die zerlegten ersten und zweiten Bilder 1-Level zerlegte Bilder werden; und

k) Schätzen der Bewegung des Zielobjektes basierend auf den gespeicherten Verschiebungen.

**2.** Verfahren nach Anspruch 1, wobei die Vielzahl von aufeinanderfolgenden Bildern Ultraschallbilder sind.

**3.** Verfahren nach Anspruch 1, wobei die Schritte b) und i) mit Wavelet-Transformation bzw. inverser Wavelet-Transformation durchgeführt werden.

**4.** Verfahren nach Anspruch 1, wobei der Schritt d) die folgenden Schritte aufweist:

d1) Berechnen von horizontalen und vertikalen Gradienten für jeden das zweite Unterbild aufweisenden Pixel;
d2) Berechnen von Gradienten von jedem Pixel des zweiten Unterbilds; und
d3) Vergleichen von Gradienten von Pixeln miteinander und Auswählen eines den maximalen Gradienten aufweisenden Pixels als das Merkmalspixel.

**5.** Verfahren nach Anspruch 4, wobei die horizontalen und vertikalen Gradienten unter Verwendung von Graustufen von jedem Pixel berechnet werden.

**6.** Verfahren nach Anspruch 4, wobei die horizontalen und vertikalen Gradienten unter Verwendung von Lumineszenz von jedem Pixel berechnet werden.

**7.** Verfahren nach Anspruch 4, wobei der Referenzbereich eine Mindestbetrags-Absolutdifferenz innerhalb des Bildblocks aufweist.

**8.** Verfahren nach Anspruch 1, wobei der Schritt g) die folgenden Schritte aufweist:

g1) Berechnen von Abstandsverschiebungen zwischen jedem Pixel des Bildblocks und jedem Pixel des Referenzbereichs; und
g2) Berechnen einer Rotationsverschiebung durch Rotieren des Bildblocks für den Referenzbereich.

**9.** Vorrichtung zur Schätzung einer Bewegung eines Zielobjekts aus einer Vielzahl von nacheinander eingegebenen Bildern, welches Folgendes aufweist:

eine erste Auswähleinheit zum Auswählen aufeinanderfolgender erster und zweiter Bilder aus der Vielzahl von Bildern;
eine Zerlegungseinheit zum Zerlegen der ersten und zweiten Bilder in eine Vielzahl von Unterbilder basierend auf den Frequenzkomponenten der ersten und zweiten Bilder jeweils in n Stufen, wobei n eine positive ganze Zahl ist;
eine zweite Auswahleinheit zum Auswählen von ersten und zweiten Unterbildern mit niedrigen Frequenzkomponenten aus der Vielzahl von Unterbildern entsprechend den ersten und zweiten Bildern;
eine Festlegeeinheit zum Festlegen eines Merkmalspixels in dem zweiten Unterbild;
eine dritte Auswahleinheit zum Auswählen eines Bildblocks, welcher das Merkmalspixel und eine vorbestimmte Anzahl von zu dem Merkmalspixel benachbarten Pixeln beeinhaltet;
eine vierte Auswahleinheit zum Auswählen eines Referenzbereichs aus dem ersten Unterbild durch Vergleichen des Bildblocks mit dem ersten Unterbild;
eine Berechnungseinheit zum Berechnen von Verschiebungen zwischen Pixeln des Referenzbereichs und Pixeln des Bildblocks;
eine Speichereinheit zum Speichern der berechneten Verschiebungen;
eine Verknüpfungseinheit zur 1 -Level-Verknüpfung der zerlegten ersten und zweiten Bilder;
eine Einheit zur wiederholten Verwendung der zweiten Auswahleinheit, der Festlegeeinheit, der dritten Auswahleinheit, der vierten Auswahleinheit, der Berechnungseinheit, der Speichereinheit und der Verknüpfungseinheit, bis die zerlegten ersten und zweiten Bilder 1-Level zerlegte Bilder werden; und
eine Schätzeinheit zum Schätzen der Bewegung des Zielobjekts basierend auf den gespeicherten Verschiebungen.

**10.** Vorrichtung nach Anspruch 9, wobei die Vielzahl von aufeinanderfolgenden Bildern Ultraschallbilder sind.

**11.** Vorrichtung nach Anspruch 9, wobei die Zerlegeeinheit und die Verknüpfungseinheit unter Verwendung von Wavelet-Transformation bzw. inverser Wavelet-Transformation betrieben werden.

**12.** Vorrichtung nach Anspruch 9, wobei die Festlegeeinheit Folgendes aufweist:

8

eine erste Berechnungseinheit zum Berechnen von horizontalen und vertikalen Gradienten für jeden das zweite Unterbild aufweisenden Pixel,

eine zweite Berechnungseinheit zum Berechnen von Gradienten von jedem Pixel des zweiten Unterbilds unter Verwendung der berechneten vertikalen und horizontalen Gradienten; und

eine Vergleichseinheit zum Vergleichen von Gradienten von Pixeln miteinander und Auswählen eines den maximales Gradienten aufweisenden Pixels als das Merkmalspixel.

13. Vorrichtung nach Anspruch 12, wobei die horizontalen und vertikalen Gradienten unter Verwendung von Graustufen von jedem Pixel berechnet werden.

14. Verfahren nach Anspruch 12, wobei die horizontalen und vertikalen Gradienten unter Verwendung von Lumineszenz von jedem Pixel berechnet werden.

15. Verfahren nach Anspruch 12, wobei der Referenzbereich eine Mindestbetrags-Absolutdifferenz innerhalb des Bildblocks aufweist.

16. Vorrichtung nach Anspruch 9, wobei die Berechnungseinheit Folgendes aufweist:

eine erste Berechnungseinheit zum Berechnen von Abstandsverschiebungen von Koordinaten zwischen jedem Pixel des Bildblocks und jedem Pixel des Referenzbereichs; und

eine zweite Berechnungseinheit zum Berechnen einer Rotationsverschiebung von Koordinaten durch Rotieren des Bildblocks für den Referenzbereich.

**Revendications**

1. Procédé d'estimation d'un mouvement d'un objet cible à partir d'une pluralité d'images entrées consécutivement, comprenant :

a) la sélection des première et seconde images consécutives parmi une pluralité d'images,

b) la décomposition des première et seconde images en une pluralité de sous-images sur la base des composantes de fréquence des première et seconde images, respectivement en $n$ niveaux, où $n$ est un entier positif,

c) la sélection de première et seconde sous-images de composantes à basse fréquence parmi la pluralité de sous-images correspondant aux première et seconde images,

d) la définition d'un pixel caractéristique dans la seconde sous-image,

e) la sélection d'un bloc d'images contenant le pixel caractéristique et un nombre prédéterminé de pixels voisins du pixel caractéristique,

f) la sélection d'une région de référence dans la première sous-image par comparaison du bloc d'images avec la première sous-image,

g) le calcul des déplacements entre des pixels de la région de référence et des pixels du bloc d'images,

h) le stockage des déplacements calculés,

i) l'exécution d'une composition de niveau 1 pour les première et seconde images décomposées,

j) l'exécution répétitive des étapes c) à i) jusqu'à ce que les première et seconde images décomposées deviennent des images décomposées de niveau 1, et

k) l'estimation du mouvement de l'objet cible sur la base des déplacements stockés.

2. Procédé tel qu'énoncé dans la revendication 1, dans lequel la pluralité d'images consécutives est constituée par des images ultrasonores.

3. Procédé tel qu'énoncé dans la revendication 1, dans lequel les étapes b) et i) sont exécutées respectivement à l'aide d'une transformée en ondelettes et à l'aide d'une transformée en ondelettes inversée.

4. Procédé tel qu'énoncé dans la revendication 1, dans lequel l'étape d) comprend les étapes de :

d1) calcul du gradient horizontal et du gradient vertical de chaque pixel constituant la seconde sous-image ;

d2) calcul du gradient de chaque pixel de la seconde sous-image, et

d3) comparaison des gradients de pixels entre eux et sélection du pixel ayant le gradient maximum comme pixel caractéristique.

**5.** Procédé tel qu'énoncé dans la revendication 4, dans lequel le gradient horizontal et le gradient vertical sont calculés à l'aide du niveau de gris de chaque pixel.

**6.** Procédé tel qu'énoncé dans la revendication 4, dans lequel le gradient horizontal et le gradient vertical sont calculés à l'aide de la luminescence de chaque pixel.

**7.** Procédé tel qu'énoncé dans la revendication 4, dans lequel la région de référence a une différence absolus de somme minimale avec le bloc d'images.

**8.** Procédé tel qu'énoncé dans la revendication 1, dans lequel l'étape g) comprend :

g1) le calcul des déplacements en distance entre chaque pixel du bloc d'images et chaque pixel de la région de référence, et
g2) le calcul des déplacements en rotation par une rotation du bloc d'images pour la région de référence.

**9.** Dispositif pour l'estimation d'un mouvement d'un objet cible à partir d'une pluralité d'images entrées consécutivement, comprenant :

une première unité de sélection pour sélectionner des première et seconde images consécutives parmi une pluralité d'images,
une unité de décomposition pour décomposer les première et seconde images en une pluralité de sous-images sur la base des composantes de fréquence des première et seconde images respectivement en $n$ niveaux, où $n$ est un entier positif,
une deuxième unité de sélection pour sélectionner des première et seconde sous-images de composantes à basse fréquence parmi la pluralité de sous-images correspondant aux première et seconde images,
une unité de définition pour définir un pixel caractéristique dans la seconde sous-image,
une troisième unité de sélection pour sélectionner un bloc d'images contenant le pixel caractéristique et un nombre prédéterminé de pixels voisins du pixel caractéristique,
une quatrième unité de sélection pour sélectionner une région de référence parmi les premières sous-images par comparaison du bloc d'images avec la première sous-image,
une unité de calcul pour calculer les déplacements entre les pixels de la région de référence et les pixels du bloc d'images,
une unité de stockage pour stocker les déplacements calculés,
une unité de composition pour exécuter une composition de niveau 1 pour les première et seconde images décomposées,
une unité pour utiliser de façon répétitive la deuxième unité de sélection, l'unité de définition, la troisième unité de sélection, la quatrième unité de sélection, l'unité de calcul, l'unité de stockage et l'unité de composition jusqu'à ce que les première et seconde images décomposées deviennent des images décomposées de niveau 1, et
une unité d'estimation pour estimer le mouvement de l'objet cible sur la base des déplacements stockés.

**10.** Dispositif tel qu'énoncé dans la revendication 9, dans lequel la pluralité d'images consécutives est constituée par des images ultrasonores.

**11.** Dispositif tel qu'énoncé dans la revendication 9, dans lequel l'unité de décomposition et l'unité de composition sont activées respectivement à l'aide d'une transformée en ondelettes et d'une transformée en ondelettes inversée.

**12.** Dispositif tel qu'énoncé dans la revendication 9, dans lequel l'unité de définition comprend :

une première unité de calcul pour calculer le gradient horizontal et le gradient vertical de chaque pixel comprenant la seconde sous-image,
une seconde unité de calcul pour calculer le gradient de chaque pixel de la seconde sous-image à l'aide du gradient vertical et du gradient horizontal calculés, et
une unité de comparaison pour comparer les gradients de pixels entre eux et sélectionner un pixel ayant un gradient maximum comme pixel caractéristique.

**13.** Dispositif tel qu'énoncé dans la revendication 12, dans lequel le gradient horizontal et le gradient vertical sont calculés à l'aide du niveau de gris de chaque pixel.

**14.** Dispositif tel qu'énoncé dans la revendication 12, dans lequel le gradient horizontal et le gradient vertical sont calculés à l'aide de la luminescence de chaque pixel.

**15.** Dispositif tel qu'énoncé dans la revendication 9, dans lequel la région de référence a une différence absolue de somme minimale avec le bloc d'images.

**16.** Dispositif tel qu'énoncé dans la revendication 9, dans lequel l'unité de calcul comprend:

une première unité de calcul pour calculer les déplacements en distance de coordonnées entre chaque pixel du bloc d'images et chaque pixel de la région de référence, et
une seconde unité de calcul destinée à calculer les déplacements en rotation de coordonnées par une rotation du bloc d'images pour la région de référence.

# Fig. 1

# FIG. 2A

```
                        ┌──────────┐
                        │  Start   │
                        └──────────┘
                              │
                              ▼
┌──────────────────────────────────────────────────────────┐
│ Decomposing ultrasound images on the basis of frequency component │─────S110
└──────────────────────────────────────────────────────────┘
                              │
                              ▼
┌──────────────────────────────────────────────────────────┐
│   Calculating horizontal and vertical gradients of sub image   │─────S120
└──────────────────────────────────────────────────────────┘
                              │
                              ▼
┌──────────────────────────────────────────────────────────┐
│              Calculating gradient of each pixel              │
│       by using claculated horizontal and vertical gradients   │─────S130
└──────────────────────────────────────────────────────────┘
                              │
                              ▼
┌──────────────────────────────────────────────────────────┐
│            Selecting a feature pixel from sub image           │─────S140
└──────────────────────────────────────────────────────────┘
                              │
                              ▼
┌──────────────────────────────────────────────────────────┐
│          Setting image block based on the feature pixel      │─────S150
└──────────────────────────────────────────────────────────┘
                              │
                              ▼
┌──────────────────────────────────────────────────────────┐
│     Selecting reference region by matching image block with   │
│   search region in sub image of previously inputted image  ·  │─────S160
└──────────────────────────────────────────────────────────┘
                              │
                              ▼
┌──────────────────────────────────────────────────────────┐
│ Calculating displacements between image block and reference region │─────S170
└──────────────────────────────────────────────────────────┘
                              │
                              ▼
┌──────────────────────────────────────────────────────────┐
│                   Storing displacements                      │─────S180
└──────────────────────────────────────────────────────────┘
                              │
                              ▼
                         To S190
```

# FIG. 2B

From S180

S190

No                1-level?                Yes

To S110

S240

All image?          No

Composing the sub image by 1 level          S200

Selecting feature pixel from composed sub image          S210

Setting image block in 1-level composed sub image          S220

Selecting a reference region in 1-level composed image of previously inputted image          S230

To S160

S250

Yes

Forming ultrasound image on the basis of calculated displacements

End

# FIG. 3

| Low/Low (LL1) | High/Low (HL1) |
|---|---|
| Low/High (LH1) | High/High (HH1) |

↑ Vertical filtering

| Image of low frequency (L) | Image of high frequency (H) |
|---|---|

↑ Horizontal filtering

| Inputted image |
|---|

# FIG. 4

## FIG. 5

## FIG. 6A

|    | C0 | C1 | C2 | C3 | C4 | C5 | C6 | C7 | C8 |
|----|----|----|----|----|----|----|----|----|----|
| R0 | 1  | 1  | 1  | 1  | 0  | -1 | -1 | -1 | -1 |
| R1 | 1  | 1  | 1  | 1  | 0  | -1 | -1 | -1 | -1 |
| R2 | 1  | 1  | 1  | 1  | 0  | -1 | -1 | -1 | -1 |
| R3 | 1  | 1  | 1  | 1  | 0  | -1 | -1 | -1 | -1 |
| R4 | 1  | 1  | 1  | 1  | 0  | -1 | -1 | -1 | -1 |
| R5 | 1  | 1  | 1  | 1  | 0  | -1 | -1 | -1 | -1 |
| R6 | 1  | 1  | 1  | 1  | 0  | -1 | -1 | -1 | -1 |
| R7 | 1  | 1  | 1  | 1  | 0  | -1 | -1 | -1 | -1 |
| R8 | 1  | 1  | 1  | 1  | 0  | -1 | -1 | -1 | -1 |

610

## FIG. 6B

|    | C0 | C1 | C2 | C3 | C4 | C5 | C6 | C7 | C8 |
|----|----|----|----|----|----|----|----|----|----|
| R0 | 1  | 1  | 1  | 1  | 1  | 1  | 1  | 1  | 1  |
| R1 | 1  | 1  | 1  | 1  | 1  | 1  | 1  | 1  | 1  |
| R2 | 1  | 1  | 1  | 1  | 1  | 1  | 1  | 1  | 1  |
| R3 | 1  | 1  | 1  | 1  | 1  | 1  | 1  | 1  | 1  |
| R4 | 0  | 0  | 0  | 0  | 0  | 0  | 0  | 0  | 0  |
| R5 | -1 | -1 | -1 | -1 | -1 | -1 | -1 | -1 | -1 |
| R6 | -1 | -1 | -1 | -1 | -1 | -1 | -1 | -1 | -1 |
| R7 | -1 | -1 | -1 | -1 | -1 | -1 | -1 | -1 | -1 |
| R8 | -1 | -1 | -1 | -1 | -1 | -1 | -1 | -1 | -1 |

620

## FIG. 7A

(RP)  610

|  | x0 | x1 | x2 | x3 | x4 | x5 | x6 | x7 | x8 | x9 | x10 | x11 | x12 | x13 | x14 |
|---|---|---|---|---|---|---|---|---|---|---|---|---|---|---|---|
| y0 | 0 | 1 | 3 | 4 | 9 | 4 | 1 | 4 | 6 | 2 | 9 | 3 | 7 | 2 | 0 |
| y1 | 2 | 4 | 3 | 3 | 4 | 2 | 1 | 2 | 4 | 2 | 4 | 3 | 6 | 3 | 1 |
| y2 | 0 | 2 | 4 | 3 | 7 | 6 | 7 | 3 | 7 | 1 | 3 | 3 | 6 | 1 | 2 |
| y3 | 6 | 1 | 5 | 6 | 8 | 4 | 5 | 5 | 8 | 3 | 4 | 2 | 8 | 4 | 3 |
| y4 | 3 | 3 | 5 | 5 | (6) | 7 | 4 | 3 | 5 | 3 | 5 | 3 | 8 | 9 | 5 |
| y5 | 6 | 8 | 6 | 5 | 7 | 3 | 8 | 2 | 8 | 3 | 4 | 5 | 1 | 6 | 7 |
| y6 | 9 | 7 | 2 | 5 | 0 | 4 | 5 | 3 | 8 | 3 | 3 | 7 | 4 | 4 | 4 |
| y7 | 1 | 6 | 4 | 1 | 1 | 4 | 5 | 5 | 9 | 7 | 4 | 8 | 5 | 4 | 6 |
| y8 | 2 | 9 | 3 | 1 | 4 | 4 | 3 | 7 | 2 | 6 | 4 | 9 | 4 | 6 | 8 |
| y9 | 5 | 2 | 3 | 2 | 6 | 2 | 6 | 4 | 3 | 8 | 4 | 5 | 5 | 8 | 6 |
| y10 | 6 | 5 | 3 | 6 | 3 | 8 | 4 | 3 | 6 | 8 | 5 | 5 | 9 | 4 | 5 |
| y11 | 9 | 7 | 6 | 3 | 2 | 4 | 3 | 5 | 4 | 9 | 6 | 4 | 7 | 3 | 5 |
| y12 | 3 | 3 | 8 | 3 | 8 | 5 | 3 | 4 | 5 | 5 | 9 | 5 | 3 | 2 | 5 |
| y13 | 6 | 1 | 8 | 8 | 8 | 2 | 2 | 2 | 8 | 6 | 9 | 5 | 1 | 1 | 2 |
| y14 | 0 | 2 | 6 | 9 | 8 | 3 | 6 | 6 | 7 | 8 | 9 | 5 | 0 | 0 | 6 |

# FIG. 7B

(RP)        620

|     | x0 | x1 | x2 | x3 | x4 | x5 | x6 | x7 | x8 | x9 | x10 | x11 | x12 | x13 | x14 |
|-----|----|----|----|----|----|----|----|----|----|----|-----|-----|-----|-----|-----|
| y0  | 0  | 1  | 3  | 4  | 9  | 4  | 1  | 4  | 6  | 2  | 9   | 3   | 7   | 2   | 0   |
| y1  | 2  | 4  | 3  | 3  | 4  | 2  | 1  | 2  | 4  | 2  | 4   | 3   | 6   | 3   | 1   |
| y2  | 0  | 2  | 4  | 3  | 7  | 6  | 7  | 3  | 7  | 1  | 3   | 3   | 6   | 1   | 2   |
| y3  | 6  | 1  | 5  | 6  | 8  | 4  | 5  | 5  | 8  | 3  | 4   | 2   | 8   | 4   | 3   |
| y4  | 3  | 3  | 5  | 5  | (6)| 7  | 4  | 3  | 5  | 3  | 5   | 3   | 8   | 9   | 5   |
| y5  | 6  | 8  | 6  | 5  | 7  | 3  | 8  | 2  | 8  | 3  | 4   | 5   | 1   | 6   | 7   |
| y6  | 9  | 7  | 2  | 5  | 0  | 4  | 5  | 3  | 8  | 3  | 3   | 7   | 4   | 4   | 4   |
| y7  | 1  | 6  | 4  | 1  | 1  | 4  | 5  | 5  | 9  | 7  | 4   | 8   | 5   | 4   | 6   |
| y8  | 2  | 9  | 3  | 1  | 4  | 4  | 3  | 7  | 2  | 6  | 4   | 9   | 4   | 6   | 8   |
| y9  | 5  | 2  | 3  | 2  | 6  | 2  | 6  | 4  | 3  | 8  | 4   | 5   | 5   | 8   | 6   |
| y10 | 6  | 5  | 3  | 6  | 3  | 8  | 4  | 3  | 6  | 8  | 5   | 5   | 9   | 4   | 5   |
| y11 | 9  | 7  | 6  | 3  | 2  | 4  | 3  | 5  | 4  | 9  | 6   | 4   | 7   | 3   | 5   |
| y12 | 3  | 3  | 8  | 3  | 8  | 5  | 3  | 4  | 5  | 5  | 9   | 5   | 3   | 2   | 5   |
| y13 | 6  | 1  | 8  | 8  | 8  | 2  | 2  | 2  | 8  | 6  | 9   | 5   | 1   | 1   | 2   |
| y14 | 0  | 2  | 6  | 9  | 8  | 3  | 6  | 6  | 7  | 8  | 9   | 5   | 0   | 0   | 6   |

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2327313 A